# EUROPEAN PATENT SPECIFICATION

(11) **EP 4 126 183 B1**
(45) Date of publication and mention of the grant of the patent: **16.07.2025**
(21) Application number: 21711053.5
(22) Date of filing: 10.03.2021
(51) Int. Cl.: F16K 5/02, F16K 11/083, F16K 27/06, A61M 39/22

(54) **IMPROVED THREE-WAY MEDICAL CONNECTOR WITH A WASHING BULKHEAD**
VERBESSERTER MEDIZINISCHER DREIWEGVERBINDER MIT WASCHSCHOTT
ÉLÉMENT DE LIAISON MÉDICAL À TROIS VOIES AMÉLIORÉ COMPRENANT UNE CLOISON DE LAVAGE

(30) Priority: 27.03.2020 IT 202000006508
(43) Date of publication of application: 08.02.2023
(73) Proprietor: GVS S.p.A., 40069 Zola Predosa (Bologna) (IT)
(72) Inventor: BETTINI, Emanuele, 40050 Monte San Pietro (BO) (IT); SCAGLIARINI, Nicola, 40136 Bologna (IT)
(74) Representative: Ripamonti, Enrico
(86) International application number: PCT/IB2021/051984
(87) International publication number: WO 2021/191715

(56) References cited:
- US-A1- 2018 035 937
- US-A1- 2018 296 820
- US-B1- 6 779 560

## Description

The subject of the present invention is a three-way medical connector according to the preamble of the main claim.

As is known, a three-way medical connector or simply three-way connector (or stopcock) is an auxiliary product which is used in lines or with systems for supply of fluid to, or collection of blood from, a patient. For example, a connector of this type is used in an administration line in order to introduce a drug therein by means of use of a syringe for example. Or, the connector is connected to (or is part of) a catheter, and through it, it is possible not only to administer a drug to the patient, but also to collect his blood.

This connector can also be part of a unit or kit for supply of a fluid endovenously, and which regulates the distribution of the aforementioned fluid to the vascular system of a patient, such as a supply kit used in the administration of drugs, for graduation in the aforementioned supply, for oncological supplies, and for contrast, nutritional and other liquids.

A known three-way connector generally comprises a body with a tubular central part, usually with an overall cylindrical form, with an inner cavity. This cavity is open at a first end of this body, and is closed at a second end thereof.

The inner cavity of the aforementioned tubular central part communicates with inner ducts of connection portions which are usually in the form of three arms which project radially from this central part of the connector. Two of these connection portions (for input) can be connected to means for supply of fluids: in general, a first input portion can be connected to a supply of a physiological solution (for example a bag which is connected by means of a tube to the connector), whereas the second input connection portion receives a medical fluid, for example from a syringe or from a line for administration of a drug.

The third arm or third connection portion (for output) is on the other hand connected to the patient for example by means of a tube which conveys the fluid to the patient (which fluid is received by at least one of the input connection portions). For example, the three-way connector is connected to a tube which can transfer the fluid to be administered to the patient, or it is associated with, or is part of, a catheter which is directly supported on the body of the patient (typically on the arm).

In order to put at least one of the input connection portions selectively into communication with the output portion, a valve element is provided, which is engaged such as to rotate inside the tubular central part of the body of the connector. This valve element comprises a body which is also generally tubular or cylindrical, and projects from the first end of the aforementioned tubular central part with a portion which acts as a grip, usually having arms which project radially in order to facilitate grasping thereof.

On the outer surface of its tubular body, the valve element has a perimetrical groove which is partly annular (i.e. which affects only part of the cylindrical surface of this body, and is not closed on itself in the form of a ring), which groove can connect at least one of the input connection portions selectively to the output portion, or can intercept and close all communication between the input and output. This takes place by means of rotation of the valve element in the central part of the connector, and appropriate selective positioning of the aforementioned groove relative to said portions. It will be appreciated that this groove can be replaced by channels or the like; in all cases, the tubular body of the valve element generally comprises at least one fluid connection unit, which can permit the connection (or interruption of the connection) between at least one input connection portion and the output connection portion, and the passage of a fluid between said portions.

Amongst the primary uses of a three-way connector is that of being able to administer to the patient drugs in addition to the basic supply, by means of the second input connection portion placed between the first input connection portion and the third, output connection portion. This supply normally takes place by means of a syringe which has an end part that is engaged at the second input connection portion according to the known standards, after a safety stopper placed thereon has been removed.

In the case of a simple three-way connector, upon completion of administration of the drug from the second connection portion, a health operator removes the syringe and puts the protective stopper back onto this portion. It can easily be imagined that, in the inner volume of this portion (blind volume) delimited on the one hand by the stopper and on the other hand by the tubular body of the valve element, there remains some of the drug which has shortly beforehand been administered via the syringe, but has not yet reached the patient.

The task of cleaning again this blind volume which still contains some drug is left up to the flow of liquid (physiological solution) of the basic supply. The volume of liquid (for washing) which must pass through the three-way connector in order for the drug to be completely administered (and thus removed from the above-described blind space) is known as the "washing volume".

A three-way connector with a reduced washing volume has the advantages of less waste of drug (which often has a high cost) and of basic solution, as well as a shorter supply time.

US2018296820 relates to a blood sampling system comprising a stopcock provided with a sampling part designed to reduce or eliminate the occurrence of stagnation of fluid flowing therethrough; the system, i.e. the stopcock and sampling port device includes a housing, an elastomeric element, a cap, a handle, and a divided septum. The housing defines an internal fluid passageway having a first port, a second port, and a third port, and the divided septum is operably coupled to the housing within the third port. The septum is configured to encourage turbulence of fluid passing through the third port, thereby reducing the occurrence of stagnation of fluid flowing therethrough.

US2018035937 relates to a flushable fluid handling assembly including a housing element defining a central bore and at least first, second and third ports, the first and third ports being line connection ports and the second port being a syringe connection port. A handle element is selectably positionable relative to the housing element, at least one fluid passageway communicating between at least two of the at least first, second and third ports. The at least one fluid passageway is selectably defined by rotational positioning of the handle element relative to the housing element. A first fluid flow guide extends radially to an inner facing wall of the central bore, the first fluid flow guide being associated with the at least one fluid flow passageway and a syringe being fixedly connected to the second port, the syringe including an axially displaceable plunger.

US6779560 relates to a multiport valve comprising a casing having at least one fluid inlet port and at least one fluid outlet port, a seal disposed adjacent to each outlet port, a cylinder disposed within a central opening in the casing and having an axial channel with a channel surface and at least one depression within the channel and balls biased toward the cylinder and away from each outlet port. Rotating the cylinder such that the depression aligns with the ball forces the biased ball into the depression and away from the seal opening the outlet port allowing for fluid flow therethrough and rotating the cylinder such that the depression is misaligned with respect to the depression causes the channel surface to overcome the bias and force the ball against the seal thus closing the outlet port. The multiport valve may include a bearing assembly disposed between the valve casing and cylinder and a ball biasing member angled to point towards the cylinder and bearing assembly, providing for ease in rotation of the cylinder.

Three-way connectors provided with inner means which can facilitate the washing of the blind volume have been known for some time. These connectors are mainly of two types, i.e. according to a first type, the valve element has a form such as to facilitate the flow of the washing liquid towards the blind volume, and according to a second type, there is also a bulkhead (for washing) inside the second input connection portion.

In a connector of the first type (such as, for example, the one described in EP2808586) the valve element has its body provided at the fluid connection unit with a wall or flow deflector which can divert the flow obtained from the first input connection portion into a blind volume, thus generating the washing volume required in order to remove the residues of drug (or other bodily fluid) which are present in the blind volume. In a connector of the second type (such as the one which is the subject of US9775981 or US9016316), the flow deflector, also known as the washing deflector, is present inside the second connection portion. For example, in the aforementioned US patents, this washing bulkhead can cooperate with the flow deflector of the rotary valve element, in order to determine the washing volume required to remove whatever is present from the blind volume.

In addition, again with reference to the second type of connector, this washing bulkhead can be integral with the connector (i.e. with the second input connection portion), as in US9016316, or it can be produced separately from the connector, and subsequently connected in hot conditions, welded on, snapped on, or otherwise connected to the body of the connector (as described in US59775981).

The present invention relates to a medical connector of the second type, and with a washing bulkhead which is produced separately from the body of the connector, but is secured thereto.

The objective of the present invention is to provide a three-way connector which is improved compared with the corresponding known connectors.

In particular, the objective of the present invention is to provide a medical connector wherein a high volume of washing liquid is directed towards the blind volume when necessary.

Another objective of the invention is to provide a medical connector of the above-described type, which can however be created with known instruments and restricted costs, and which can be used in a conventional manner.

A further objective of the invention is to provide a medical connector wherein it is possible to optimise the conveying of the washing fluid towards the blind volume, such as, for example, to prevent the formation of turbulence of liquid which is stagnating inside the blind volume.

These objectives and others, which will become apparent to persons skilled in the art, are achieved by a three-way connector according to the main claim.

For better understanding of the present invention, the following drawings are appended purely by way of non-limiting example, wherein:
figure 1 shows a view in perspective of a three-way connection according to the invention;
figure 2 shows an exploded view in perspective of the connector in figure 2;
figure 3 shows a view in perspective and in cross-section, along the line 3-3 in figure 1, of the connector according to the invention, but from another angle;
figure 4 shows a view in cross-section along the line 4-4 in figure 1;
figure 5 shows a front view of the connector in figure 1;
figure 6 shows a view in cross-section along the line 6-6 in figure 5;
figure 7 shows a view in perspective of a detail of the connector in figure 1;
figure 8 shows a lateral view of the detail of the connector in figure 7;
figure 9 shows a view from below of the detail of the connector in figure 7;
figure 10 shows a front view of the detail of the connector shown in figure 7;
figure 11 shows an exploded view in perspective of a variant of the connector according to the invention;
figure 12 shows a view from above of the connector in figure 11;
figure 13 shows a view in cross-section along the line 13-13 in figure 12;
figure 14 shows a view in cross-section along the line 14-14 in figure 12;
figure 15 shows a view from above in cross-section along the line 15-15 in figure 13;
figure 16 shows a view in perspective of a component of the connector in figure 11;
figure 17 shows a lateral view of the component in figure 16;
figure 18 shows a view in perspective of another component of the connector in figure 16;
figure 19 shows a different view in perspective from another side of the component in figure 18;
figure 20 shows a lateral view of the component in figure 18; and
figures 21A-21D show partial views in perspective and in cross-section along the axis 15-15 in figure 13 of the medical connector according to the invention at various moments of use of the connector.

With reference to the aforementioned figures, a three-way connector according to the invention is generally indicated as 1. It comprises a body 2 with a tubular central part 3 which is open at a first end 4, and closed at a second end 5. The central part has a cavity 6 to which there are connected ducts 7, 8 and 9 provided respectively inside connection portions 10, 11 and 12 projecting radially from the tubular central part 3. The first and the second (input) portions 10 and 11 can be connected to corresponding supplies of fluid to be supplied to a patient, in a controlled manner, through the three-way connector, and through the third connection portion (for output of the fluid) 12. For example, the first input connection 10 can be connected to a line for supply of a physiological solution, whereas the second input connection 11 can be connected in a known manner to a syringe, for supply of a drug to a patient.

In the cavity 6 of the central part 3, there is inserted a valve element 13 having a tubular body 14, which is introduced from the first end 4 of the central part 3. The tubular body 14 has an end gripping portion 16 placed outside the cavity 6, and having radial arms 16A. Inside a surface 18 of this tubular body 14, which can face an inner surface 19 of the aforementioned cavity 6, a (semi-annular) groove 20 is provided, the function of which is to put the portions 10, 11 and 12 into selective communication and permit or prevent the supply of fluid, of fluid and a drug, or only the drug, to the patient. The body 14 is thus shaped so as to have said function.

This takes place in a manner which in itself is known to persons skilled in the art, and will therefore not be described further.

In the embodiment shown in the figures, the gripping portion 16 is perforated centrally by means of a through-hole 21 which defines a first open end of the inner cavity 16K of the valve element. This cavity 16K is also open at a second end 24 thereof, placed at the front of the second, closed end 5 of the tubular part 3 of the body 2.

A stopper 27 of a type which in itself is known can be disposed in the hole 21.

The cavity 16K of the valve unit 13 has a transverse wall 35 which subdivides this cavity into two sections, i.e. a first (upper) section 36 which communicates with the hole 21, and a second (lower) section 37 which is closed by the second end 24 of the cavity 16. In this transverse wall 35 there is present a known through-hole 40 which puts the two sections 36 and 37 of the cavity 16 into communication.

The first input connection portion 10 is provided on an outer surface thereof with fins 43 which can allow it to be connected to a tube of a medical line (which is connected for example to a bag of physiological solution), not shown. The fins define a (partial, as in the figures) or complete thread.

Similar fins 44 and 45 are present on the exterior of the second input connection portion 11 and of the output connection portion 12; these fins can cooperate by screwing respectively and alternatively with closure bodies and connection bodies. For example, the fins 44 cooperate with a closure body or stopper 46 which can reclose the second input connection portion 11 when it is not being used, whereas the fins 45 can cooperate with a tubular body 47 which is threaded in the interior, and for example can tighten on the output connection portion 12 a tube which is directed to a patient; in this last case, the body 47 is engaged on an outer connection body, which body is typically connected to a tube directed to the patient (not shown).

In the case of the second connection portion 11, instead of the above-described closure stopper, a syringe for introduction of a drug into the portion 11 can be connected to the fins 44.

Preferably, the stopper 46 can be provided with parts which can yield and/or be opened, in order to permit the passage of a needle of a syringe in order to inject into the connector 1 a drug that is then conveyed by the fluid, which enters through the first connection portion 10, and exits from the third connection portion 12, towards the patient.

At least partly inside the duct 8 (and also inside the second connection portion 11) there is present a unit 50 which acts as a deflector for the flow of fluid which reaches the first input connection portion 10, and directs the flow into the second connection portion 11, in order to remove the medical liquid which is present in the blind volume of this second portion delimited by the cavity 6 of the central part 3 of the body 2 of the connector, and by the stopper 46 which recloses the second connection portion 11 when it does not need to be used in order to insert a drug in the connector.

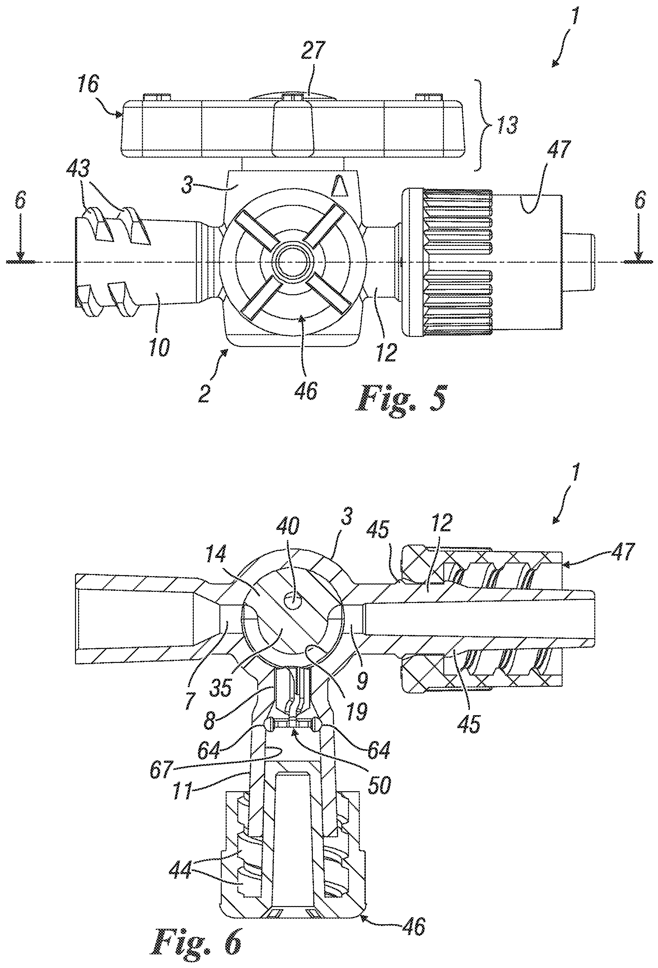

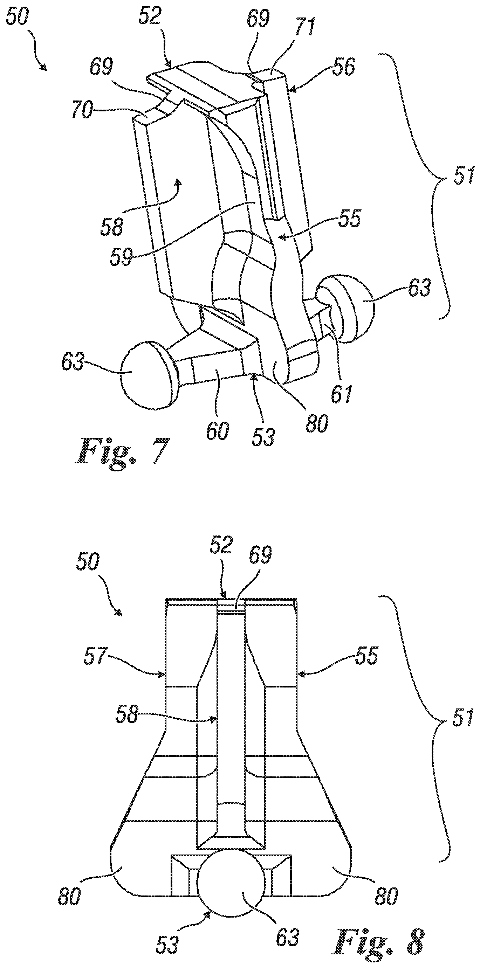

More particularly, with reference to figures 6 to 10, the unit or deflector 50 comprises a body 51 with a first end 52 and a second end 53. The body 51 is inserted axially (i.e. along the longitudinal axis) inside the duct 8, but its first end 52 projects into the cavity 6 of the body 2 of the connector (at the groove 20 provided in the tubular body 14 of the valve element 13). From this first end 52, there extend towards the second end 53 four walls 55-58 disposed substantially orthogonally to one another and longitudinally along the body 51.

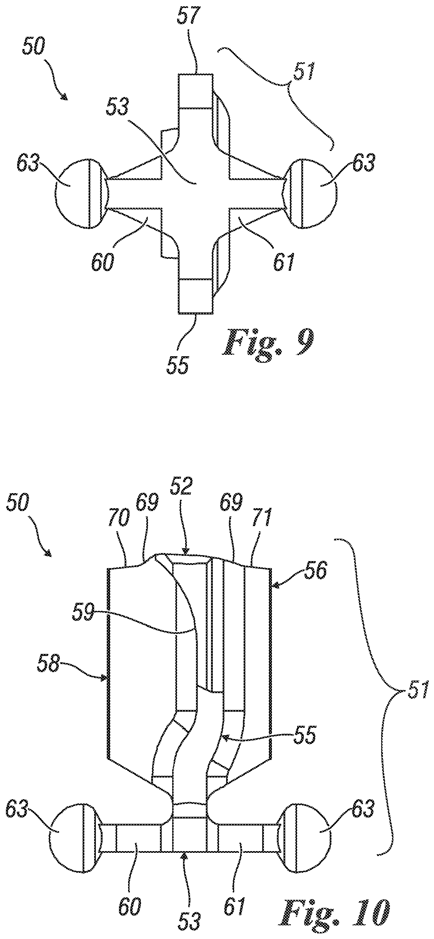

More particularly, the first and third walls 55, 57 or "diverting walls", which are opposite one another, are disposed orthogonally to the flow of the fluid in the groove 20 of the valve element 13. These walls 55 and 57 have a (slightly) arched surface, with spokes such as to "close" towards the first connection portion 10. In other words, as can be seen in figure 10, the first and third diverting walls have a surface which is concave, or with concavity 59 facing in the direction of input of the fluid into the first input connection portion. Thanks to this form, and to the fact that the first end 52 of the body of the deflector 51 penetrates (slightly) into the groove 20 of the valve element 13, when a fluid enters the first connection portion 10 and goes into the groove 20 in order to reach the output connection portion 12, this flow is intercepted substantially by the diverting walls 55 and 57 (orthogonal to the aforementioned flow), and is directed (or diverted) as a washing volume into the duct 8, and then into the second input connection portion 11. This washing liquid or volume goes into said second connection portion and exits therefrom (since the portion is closed by the stopper 46), still moving along the diverting walls 55 and 57, but on the side opposite the concave surface 59, in order to go into the output connection portion 12.

Thanks to the projection of the first end 51 into the body 3 and into the groove 20, a relatively substantial quantity of fluid is intercepted while it is flowing towards the output connection portion 12, thus ensuring optimal washing of the blind volume and optimal safety for the operators who are operating on the patient, as well as for the patient himself (who are thus not at risk of coming into contact with potentially dangerous fluids).

The other two walls 56 and 58 of the body 51 of the deflector 50 are placed orthogonally to the above-described diverting walls 55 and 57, and act as separators for the fluid input into the duct 8, which by this means can "wash" the blind volume in an optimal manner thanks to the diverting walls 55 and 57, and can flow better out of the duct 8 towards the duct 9 of the output connection portion 12.

At the end 53, the body 51 of the deflector 50 comprises opposite projecting arms 60 and 61 which are placed below the walls 56 and 58, and are resiliently deformable. These arms preferably have a triangular form, and taper at the free end, which provides them with rigidity in the direction orthogonal thereto.

Each arm supports an element which is semi-spherical, or (at least) has a spherical end cap 63 which can be snapped into seats 64 which are radiated or have a curvilinear surface provided inside an inner wall 67 of the second output connection portion 11.

This form of the end elements 63 and of the corresponding seats facilitates the insertion of the deflector 50 inside the duct 8, and its retention in position. A contribution in this respect is also made by a triangular area 80 of the diverting walls 55 and 57, which area is supported on the wall 67 of the connection portion 11, said triangular area 80 being close to the second end 53 of the body 51 of the deflector 50.

It should be noted that the first end 52 of the body 51 of the deflector 50 has arched surfaces 69 for connection with the ends 70 and 71 of the walls 56 and 58. Each of these surfaces can cooperate with a corresponding end part (not shown) of the groove 20 of the tubular body 14 of the valve element 13, when it rotates in the cavity 6 of the body 2 of the connector 1, in order to form the required connection between at least one of the input portions 10, 11 and the output connection portion 12 of this body, or to interrupt this connection.

Thanks to the connection surface 69, which is arched with a cavity facing towards the cavity 6, the body 14 of the valve element 13 can rotate freely in the aforementioned cavity 6. When it meets the first end 52 of the body 51 of the deflector 50, this body 14 "slides" on the adjacent connection surface 69, thus thrusting the deflector 50 towards the interior of the duct 8 and of the second input connection portion 11. Thanks to the resilience of the arms 60 and 61 and the insertion of their semi-spherical elements 63 into the respective seats 64, displacement of the body 51 of the deflector 50 takes place towards the interior of said connection portion 11, and the body 14 of the valve element 13 can rotate freely in the cavity 6 of the body of the connector 2. When the rotation is completed, the resilient arms 60 and 61 take the body 51 back into position in the duct 8, with the first end 51 inserted in the cavity 6, and in particular in the groove 20 of said valve element.

The invention thus makes it possible to direct a quantity of washing volume towards the second connection portion 11 without any modification in the production of the valve element (which need not be specific: in order to obtain the washing of the blind volume). This washing volume is created by collecting the fluid which penetrates into the groove 20 directly from the groove (thanks to the first end 52 of the deflector 50 projecting into the groove 20) and directing the fluid into the aforementioned connection portion thanks to the deflector 50. The deflector, which is resiliently connected (by being snapped in or also welded by ultrasound or by means of another method) to the wall 67 of the second connection portion 11, permits normal movement of the valve element in the cavity 6 of the body of the connector 2.

A description has been provided of a specific embodiment of the invention. However, on the basis of the foregoing description, persons skilled in the art can obtain other solutions, such as the one wherein the deflector 50 comprises only two opposite diverting walls 55 and 57, or wherein the diverting walls 55 and 57 do not have a curvilinear development. Furthermore, the resilient arms 60 and 61 can be associated with the wall 67 without providing the semi-spherical elements 63, but, for example, by being welded or glued directly on this wall. It should be noted that the seats 64 can have a semi-spherical form, or they can be defined by a single groove which is toroidal (or axisymmetric) provided in the wall 67 of the second connection portion 11. In this case, a coupling will be provided between the body 51 and this wall in order to prevent the rotation of the deflector 50 around the longitudinal axis of the duct 8 / second connection portion 11.

It should also be noted that the diverting walls 55 and 57 define sections of the duct 8 which are different because of the arched form of the surface 59. However, these sections can also have a substantially identical volume.

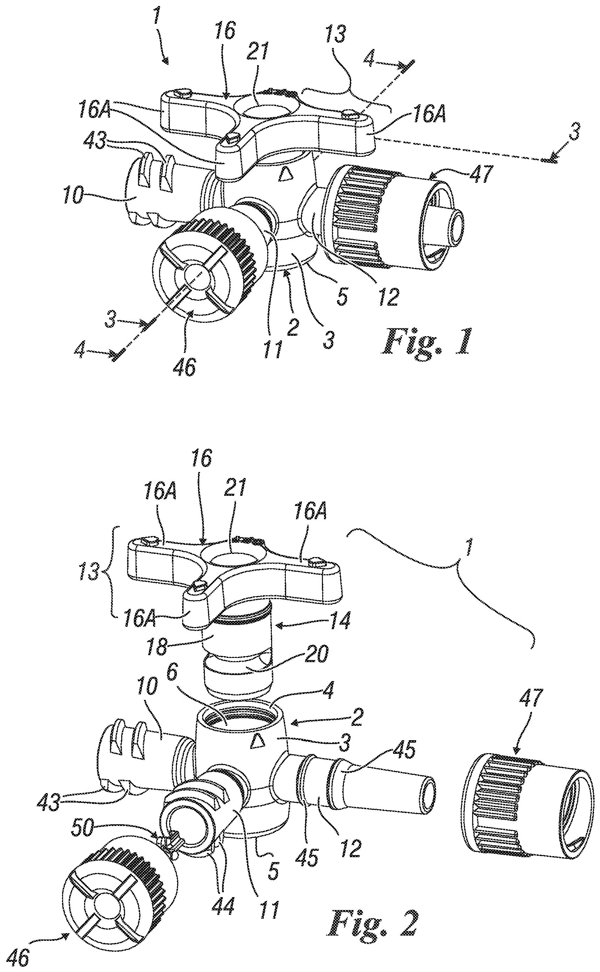

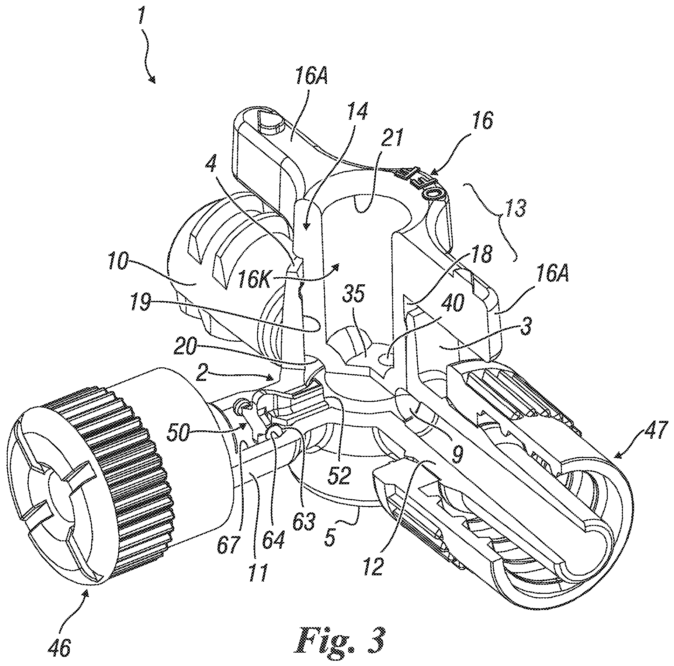

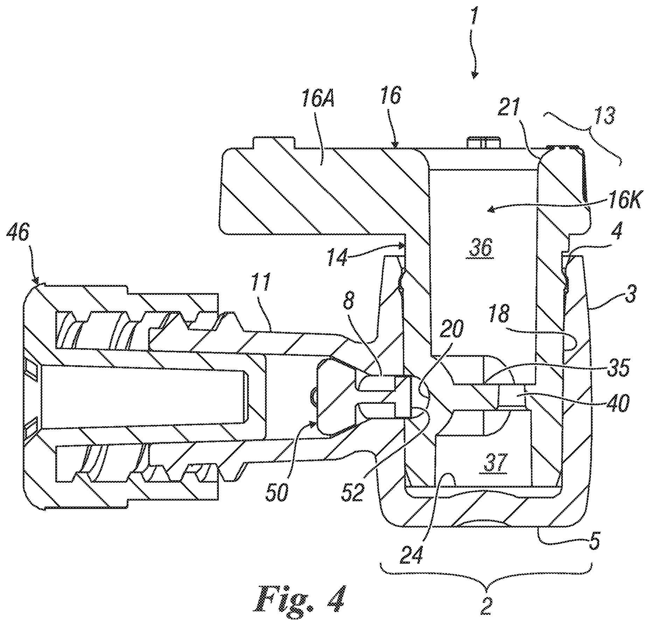

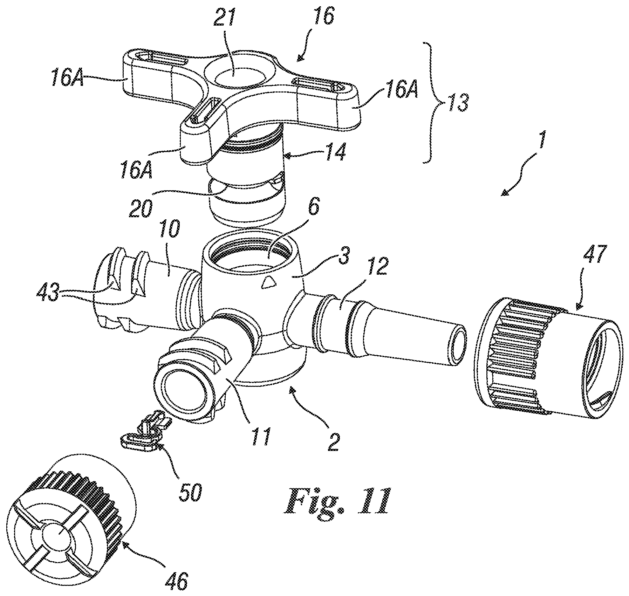

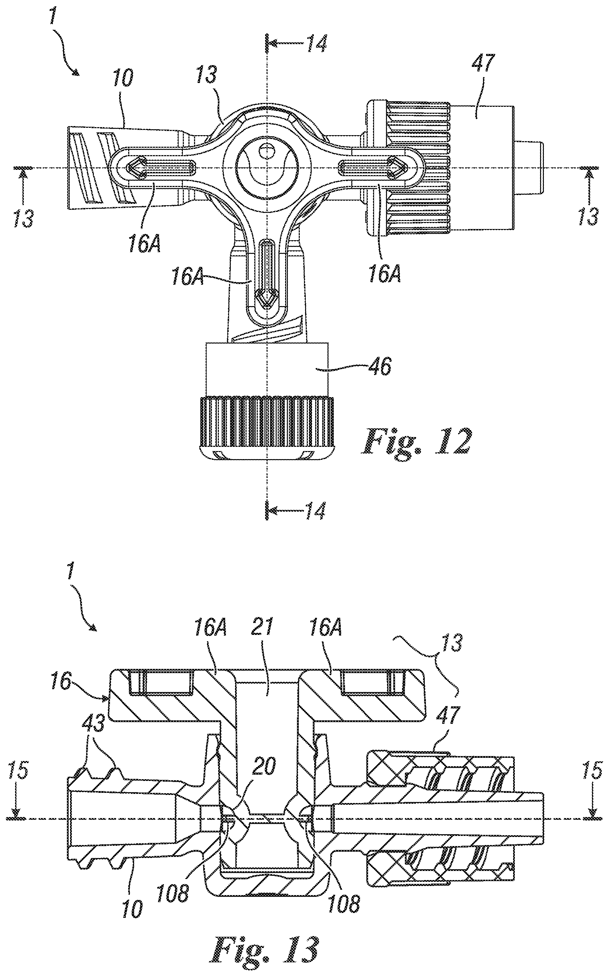

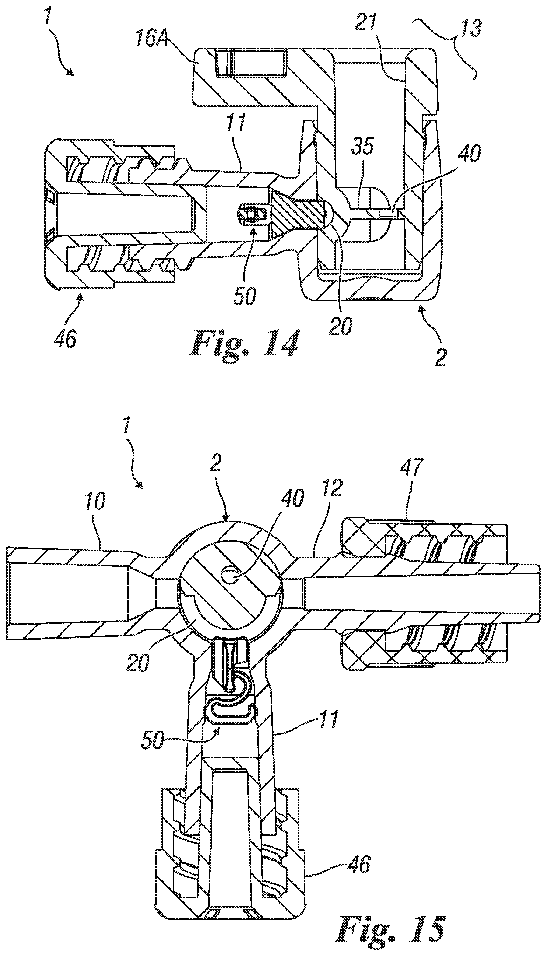

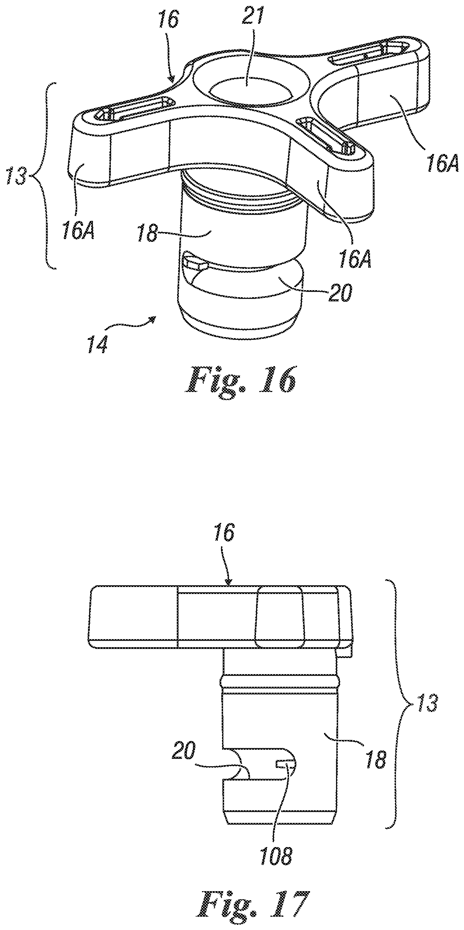

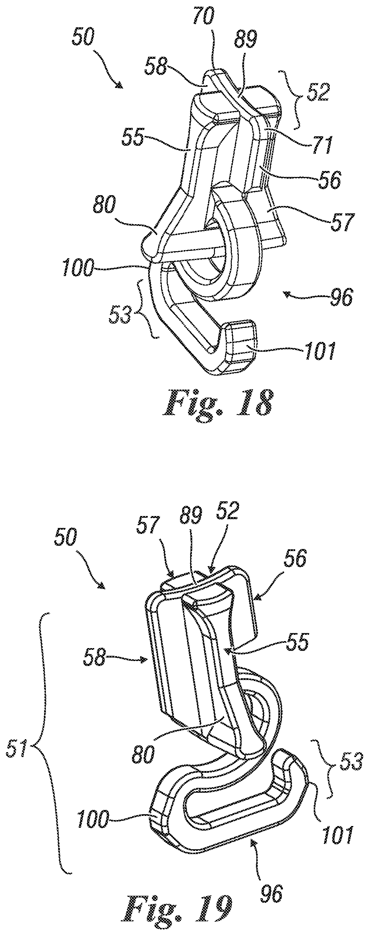

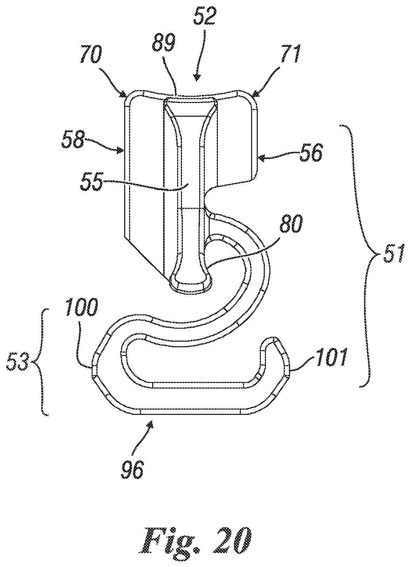

Figures 11-21D show a variant of the invention. In these figures, parts corresponding to those already described are indicated with the same numerical references as the corresponding parts described with reference to figures 1-10.

In the figures in question, the deflector unit has the body 51 provided with the first end 52 and the second end 53. However, in this embodiment of the invention, the first end has a surface 89 which is continuously concave above the walls 56 and 58, i.e. it is raised at the ends 70 and 71 of these walls. In addition, there is a resiliently deformable element, substantially in the form of an "S" or a serpentine shape 96 at the end 53, with opposite end edges 100 and 101 which are arched but not curvilinear (i.e. they are broken) (corresponding to the above-described spherical caps 63), which edges can cooperate with the seats 64 similarly to what takes place in the case of the aforementioned caps 63.

This (resilient) element 96 permits the movement of the deflector unit 50 towards the interior of the connection portion 11, when the valve element 13 is rotated.

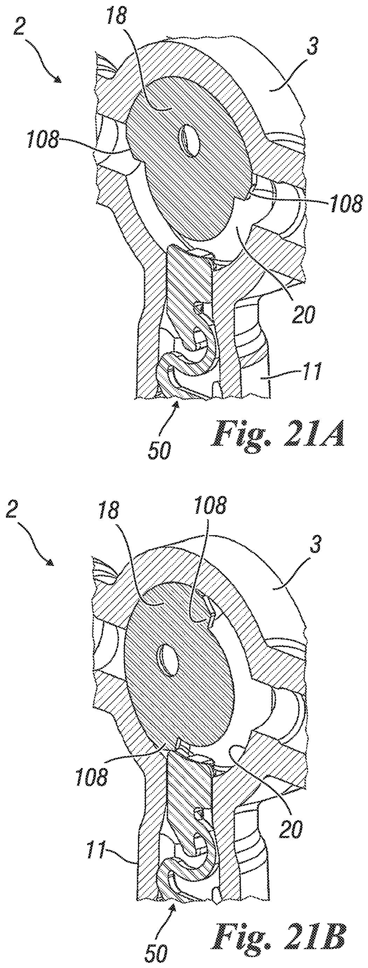

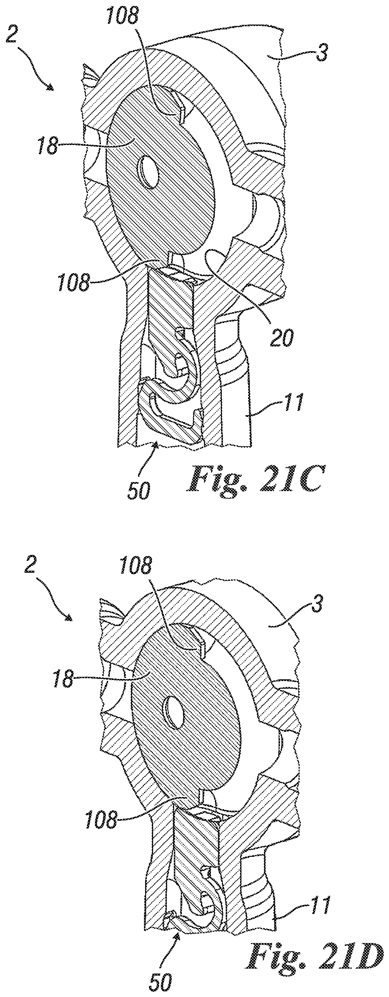

The movement in particular is generated by bosses 108 projecting inside the groove 20, which, with the rotation of the valve element 13, come into contact with the adjacent ends 70 and 71 of the wall 56 or 58, and, by pressing on the end itself, displace the deflector towards the interior of the connection portion 11 (see figures 21A-21D).

In fact, in this case also, when at rest, the first end 52 of the body 51 of the deflector unit 50 projects into the groove 20, such as to direct a quantity of washing volume towards the portion 11.

Thanks to the fact that the deflector unit 50 is separate from the body 2 of the connector 1, the invention makes it possible to form said unit differently, such as to optimise the divergence of the flow of the fluid defining the washing volume, and avoiding turbulence of liquid stagnating inside the blind volume.

These solutions also must be considered to be included in the scope of the invention as defined by the following claims.

## Claims

1. Three-way medical connector (1) comprising a body (2) having a tubular central part (3) from which connection portions (10, 11, 12) project radially, a first (10) and a second (11) input connection portion being able to receive fluids, at least one of these fluids being administered to a patient through a third, output connection portion (12), inside a cavity (6) of said tubular central part (3) there being disposed such as to rotate a tubular valve element (13) with a perimetrical groove (20) which can put at least one out of said first and second connection portions (10, 11) selectively into communication with the third connection portion (12), inside said second connection portion (11) there being provided a washing bulkhead or flow deflector (50) which can direct a washing fluid obtained from the first connection portion (10) into said second connection portion (11), said washing bulkhead or deflector (50) being an autonomous element relative to the body (2) of the connector (1), **characterised in that** the deflector or washing bulkhead projects into the cavity (6) of the tubular central part (3) of the body (2) of the connector (1) where the rotary tubular valve element (13) is present, said deflector (50) comprising a body (51) having a first end (52) and a second end (53), said body (51) being disposed inside said second connection portion (11) and along the longitudinal axis of this second portion (11), the first end (52) projecting into the cavity (6) of the tubular central part (3) of the body (2) of the connector and into the perimetrical groove (20) of the tubular valve element (13), the second end (53) being at least partly resiliently deformable and connected to an inner wall (67) of said second connection portion (11) so as to allow the deflector member (50) to move within the second connection portion (11).

2. Medical connector according to claim 1, **characterised in that** said second end (53) of the body (51) of the deflector (50) comprises two resiliently deformable opposite projecting arms (60, 61) which are connected to the inner wall (67) of the second connection portion (11).

3. Medical connector according to claim 1, **characterised in that** said second end (53) of the body (51) of the deflector (50) comprises a resiliently deformable serpentine element (96) with broken opposite arched edges (100, 101) connected to the inner wall (67) of the second connection portion (11).

4. Medical connector according to claim 1, **characterised in that** said deflector (50) is at least partly placed inside a duct (8) of the second connection portion (11), which duct is connected to the cavity (6) of the tubular central part (3) of the body (2) of the connector (1).

5. Medical connector according to claims 1 and 2 or 1 and 3, **characterised in that** the deflector (50) is coupled mechanically by being snapped in, or alternatively it is welded or glued on the inner wall (67) of the second connection portion (11).

6. Medical connector according to claim 2, **characterised in that** each projecting arm (60, 61) comprises an end semi-spherical element (63) inserted inside a corresponding seat which is radiated or has a curvilinear surface (64) provided in the inner wall (67) of the second connection portion (11).

7. Medical connector according to claim 1, **characterised in that** said body (51) of the deflector comprises diverting walls (55, 57) which are disposed longitudinally along this body (51), which walls can project inside the cavity (6) of the tubular central part (3) of the body (2) of the connector (1) and are disposed orthogonally to the flow of fluid into the perimetrical groove (20) of the tubular valve element (13), said walls (53, 57) diverting this flow into the second connection portion (11).

8. Medical connector according to claim 7, **characterised in that** said diverting walls (55, 57) placed orthogonally to the flow of the fluid have an arched or concave surface (59) with concavity facing in the direction of input of the fluid into the first connection portion (10).

9. Medical connector according to claim 7, **characterised in that** said body (51) of the deflector comprises additional walls (56, 58) which are orthogonal to said diverting walls disposed orthogonally to the flow of fluid into the perimetrical groove (20) of said tubular valve element (13).

10. Medical connector according to claim 9, **characterised in that** said body (51) has an arched connection surface (69, 89) at its first end (52) and between the ends (70, 71) of the additional walls (56, 58) perpendicular to the diverting walls (55, 57) orthogonal to the flow of the fluid into the perimetrical groove (20) of the tubular valve element (13).

11. Medical connector according to claim 10, **characterised in that** said arched surface (69) at the first end (52) of the body (51) of the deflector (50) can cooperate with end walls of the perimetrical groove (20) of the tubular valve element (13), said cooperation generating a force on said body (51) which can displace it resiliently inside the second connection portion (11), moving the body away or making it exit from the cavity (6) of the tubular central part (3) of the connector body (2).

12. Medical connector according to claim 10, **characterised in that** said arched surface (89) at the first end (52) of the body (51) of the deflector (50) can cooperate with bosses (108) which are present inside the perimetrical groove (20) of the tubular valve element (13).

13. Medical connector according to claim 7, **characterised in that** the diverting walls (55, 57) which are disposed orthogonally to the flow of the fluid into the perimetrical groove (20) of said tubular valve element (13) have a triangular area (80) at the second end (53) of the body of the deflector.

## Patentansprüche

1. Medizinischer Dreiwegverbinder (1), umfassend einen Körper (2), der ein rohrförmiges Mittelteil (3) aufweist, von dem Verbindungsabschnitte (10, 11, 12) radial hervorstehen, einen ersten (10) und einen zweiten (11) Eingangsverbindungsabschnitt, die in der Lage sind, Fluide aufzunehmen, wobei mindestens eines dieser Fluide einem Patienten über einen dritten Ausgangsverbindungsabschnitt (12) verabreicht wird, in einem Hohlraum (6) des rohrförmigen Mittelteils (3) ein rohrförmiges Ventilelement (13) mit einer umlaufenden Nut (20), das zumindest einen von dem ersten und dem zweiten Verbindungsabschnitt (10, 11) selektiv mit dem dritten Verbindungsabschnitt (12) in Verbindung bringen kann, angeordnet ist, um zu drehen innerhalb des zweiten Verbindungsabschnitts (11) ein Waschschott oder ein Stromdeflektor (50) bereitgestellt ist, der ein Waschfluid, das von dem ersten Verbindungsabschnitt (10) erlangt wird, in den zweiten Verbindungsabschnitt (11) leiten kann, wobei das Waschschott oder der Deflektor (50) ein eigenständiges Element in Bezug auf den Körper (2) des Verbinders (1) ist, **dadurch gekennzeichnet, dass** der Deflektor oder das Waschschott in den Hohlraum (6) des rohrförmigen Mittelteils (3) des Körpers (2) des Verbinders (1) hervorsteht, wo das drehende rohrförmige Ventilelement (13) vorhanden ist, der Deflektor (50) umfassend einen Körper (51), der ein erstes Ende (52) und ein zweites Ende (53) aufweist, wobei der Körper (51) im Inneren des zweiten Verbindungsabschnitts (11) und entlang der Längsachse dieses zweiten Abschnitts (11) angeordnet ist, wobei das erste Ende (52) in den Hohlraum (6) des rohrförmigen Mittelteils (3) des Körpers (2) des Verbinders und in die umlaufende Nut (20) des rohrförmigen Ventilelements (13) hervorsteht, wobei das zweite Ende (53) zumindest teilweise elastisch verformbar und mit einer Innenwand (67) des zweiten Verbindungsabschnitts (11) verbunden ist, um dem Deflektorelement (50) zu ermöglichen, sich innerhalb des zweiten Verbindungsabschnitts (11) zu bewegen.

2. Medizinischer Verbinder nach Anspruch 1, **dadurch gekennzeichnet, dass** das zweite Ende (53) des Körpers (51) des Deflektors (50) zwei elastisch verformbare, gegenüberliegende, hervorstehende Arme (60, 61) umfasst, die mit der Innenwand (67) des zweiten Verbindungsabschnitts (11) verbunden sind.

3. Medizinischer Verbinder nach Anspruch 1, **dadurch gekennzeichnet, dass** das zweite Ende (53) des Körpers (51) des Deflektors (50) ein elastisch verformbares Serpentinelement (96) mit gebrochenen, gegenüberliegenden gebogenen Rändern (100, 101) umfasst, das mit der Innenwand (67) des zweiten Verbindungsabschnitts (11) verbunden ist.

4. Medizinischer Verbinder nach Anspruch 1, **dadurch gekennzeichnet, dass** der Deflektor (50) zumindest teilweise innerhalb eines Kanals (8) des zweiten Verbindungsabschnitts (11) platziert ist, wobei der Kanal mit dem Hohlraum (6) des rohrförmigen Mittelteils (3) des Körpers (2) des Verbinders (1) verbunden ist.

5. Medizinischer Verbinder nach einem der Ansprüche 1 und 2 oder 1 und 3, **dadurch gekennzeichnet, dass** der Deflektor (50) mechanisch durch Einrasten mit der Innenwand (67) des zweiten Verbindungsabschnitts (11) gekoppelt oder alternativ daran geschweißt oder geklebt ist.

6. Medizinischer Verbinder nach Anspruch 2, **dadurch gekennzeichnet, dass** jeder hervorstehende Arm (60, 61) ein halbkugelförmiges Endelement (63) umfasst, das in einen entsprechenden Sitz eingesetzt ist, der ausgestrahlt ist oder eine gekrümmte Oberfläche (64) aufweist, die in der Innenwand (67) des zweiten Verbindungsabschnitts (11) bereitgestellt ist.

7. Medizinischer Verbinder nach Anspruch 1, **dadurch gekennzeichnet, dass** der Körper (51) des Deflektors Ablenkwände (55, 57) umfasst, die in Längsrichtung entlang dieses Körpers (51) angeordnet sind, wobei die Wände in den Hohlraum (6) des rohrförmigen Mittelteils (3) des Körpers (2) des Verbinders (1) hervorstehen können und orthogonal zu dem Fluidstrom in die umlaufende Nut (20) des rohrförmigen Ventilelements (13) angeordnet sind, wobei die Wände (53, 57) diesen Strom in den zweiten Verbindungsabschnitt (11) ablenken.

8. Medizinischer Verbinder nach Anspruch 7, **dadurch gekennzeichnet, dass** die Ablenkwände (55, 57), die orthogonal zu dem Strom des Fluids angeordnet sind, eine gewölbte oder konkave Oberfläche (59) mit einer Konkavität aufweisen, die in die Eintrittsrichtung des Fluids in den ersten Verbindungsabschnitt (10) weist.

9. Medizinischer Verbinder nach Anspruch 7, **dadurch gekennzeichnet, dass** der Körper (51) des Deflektors zusätzliche Wände (56, 58) umfasst, die orthogonal zu den Ablenkwänden sind, die orthogonal zu dem Strom des Fluids in die umlaufende Nut (20) des rohrförmigen Ventilelements (13) angeordnet sind.

10. Medizinischer Verbinder nach Anspruch 9, **dadurch gekennzeichnet, dass** der Körper (51) an seinem ersten Ende (52) und zwischen den Enden (70, 71) der zusätzlichen Wände (56, 58) senkrecht zu den Ablenkwänden (55, 57) orthogonal zu dem Strom des Fluids in die umlaufende Nut (20) des rohrförmigen Ventilelements (13) eine gewölbte Verbindungsfläche (69, 89) aufweist.

11. Medizinischer Verbinder nach Anspruch 10, **dadurch gekennzeichnet, dass** die gewölbte Fläche (69) an dem ersten Ende (52) des Körpers (51) des Deflektors (50) mit den Endwänden der umlaufenden Nut (20) des rohrförmigen Ventilelements (13) zusammenwirken kann, wobei das Zusammenwirken eine Kraft auf den Körper (51) erzeugt, die ihn elastisch innerhalb des zweiten Verbindungsabschnitts (11) verschieben kann, wodurch der Körper wegbewegt oder aus dem Hohlraum (6) des rohrförmigen Mittelteils (3) des Verbinderkörpers (2) herausgeführt wird.

12. Medizinischer Verbinder nach Anspruch 10, **dadurch gekennzeichnet, dass** die gewölbte Oberfläche (89) an dem ersten Ende (52) des Körpers (51) des Deflektors (50) mit Vorsprüngen (108) zusammenwirken kann, die innerhalb der umlaufenden Nut (20) des rohrförmigen Ventilelements (13) vorhanden sind.

13. Medizinischer Verbinder nach Anspruch 7, **dadurch gekennzeichnet, dass** die Ablenkwände (55, 57), die orthogonal zu dem Strom des Fluids in die umlaufende Nut (20) des rohrförmigen Ventilelements (13) angeordnet sind, an dem zweiten Ende (53) des Körpers des Deflektors eine dreieckige Fläche (80) aufweisen.

## Revendications

1. Connecteur médical à trois voies (1) comprenant un corps (2) ayant une partie centrale tubulaire (3) de laquelle des parties de connexion (10, 11, 12) font saillie radialement, une première (10) et une deuxième (11) partie de connexion d'entrée pouvant recevoir des fluides, au moins un de ces fluides étant administré à un patient à travers une troisième partie de connexion de sortie (12), dans une cavité (6) de ladite partie centrale tubulaire (3) est disposé de manière à tourner un élément de valve tubulaire (13) avec une rainure périmétrique (20) pouvant mettre au moins une desdites première et deuxième parties de connexion (10, 11) en communication sélective avec la troisième partie de connexion (12), à l'intérieur de ladite deuxième partie de connexion (11) se trouve une cloison de lavage ou un déflecteur de flux (50) permettant de diriger un fluide de lavage obtenu à partir de la première partie de connexion (10) dans ladite deuxième partie de connexion (11), ladite cloison de lavage ou ledit déflecteur (50) est un élément autonome par rapport au corps (2) du connecteur (1), **caractérisé en ce que** le déflecteur ou la cloison de lavage fait saillie dans la cavité (6) de la partie centrale tubulaire (3) du corps (2) du connecteur (1) où se trouve l'élément de valve tubulaire rotatif (13), ledit déflecteur (50) comprenant un corps (51) doté d'une première extrémité (52) et d'une deuxième extrémité (53), ledit corps (51) étant disposé à l'intérieur de ladite deuxième partie de connexion (11) et le long de l'axe longitudinal de cette deuxième partie (11), la première extrémité (52) faisant saillie dans la cavité (6) de la partie centrale tubulaire (3) du corps (2) du connecteur et dans la rainure périmétrique (20) de l'élément de valve tubulaire (13), la deuxième extrémité (53) étant au moins en partie déformable de manière élastique et reliée à une paroi intérieure (67) de ladite deuxième partie de connexion (11) de manière à permettre à l'élément déflecteur (50) de se déplacer à l'intérieur de la deuxième partie de connexion (11).

2. Connecteur médical selon la revendication 1, **caractérisé en ce que** ladite deuxième extrémité (53) du corps (51) du déflecteur (50) comprend deux bras en saillie opposés (60, 61) élastiquement déformables reliés à la paroi intérieure (67) de la deuxième partie de connexion (11).

3. Connecteur médical selon la revendication 1, **caractérisé en ce que** ladite deuxième extrémité (53) du corps (51) du déflecteur (50) comprend un élément serpentin (96) élastiquement déformables avec des bords arqués opposés cassés (100, 101) connectés à la paroi intérieure (67) de la deuxième partie de connexion (11).

4. Connecteur médical selon la revendication 1, **caractérisé en ce que** ledit déflecteur (50) est au moins en partie disposé à l'intérieur d'un conduit (8) de la deuxième partie de connexion (11), ce conduit étant connecté à la cavité (6) de la partie centrale tubulaire (3) du corps (2) du connecteur (1).

5. Connecteur médical selon les revendications 1 et 2 ou 1 et 3, **caractérisé en ce que** le déflecteur (50) est couplé mécaniquement par encliquetage, ou alternativement par soudage ou collage sur la paroi intérieure (67) de la deuxième partie de connexion (11).

6. Connecteur médical selon la revendication 2, **caractérisé en ce que** chaque bras en saillie (60, 61) comprend un élément semi-sphérique d'extrémité (63) inséré dans un siège correspondant qui est irradié ou présente une surface curviligne (64) qui se trouve dans la paroi intérieure (67) de la deuxième partie de connexion (11).

7. Connecteur médical selon la revendication 1, **caractérisé en ce que** ledit corps (51) du déflecteur comprend des parois de déviation (55, 57) disposées en longueur le long de ce corps (51), dont les parois pouvant faire saillie dans la cavité (6) de la partie centrale tubulaire (3) du corps (2) du connecteur (1) et sont disposées de façon orthogonale au flux de fluide dans la rainure périmétrique (20) de l'élément de valve tubulaire (13), lesdites parois (53, 57) déviants ce flux dans la deuxième partie de connexion (11).

8. Connecteur médical selon la revendication 7, **caractérisé en ce que** lesdites parois de déviation (55, 57) disposées de façon orthogonale au flux de fluide ont une surface arquée ou concave (59) dont la concavité est opposée à la direction d'entrée du fluide dans la première partie de connexion (10).

9. Connecteur médical selon la revendication 7, **caractérisé en ce que** le corps (51) du déflecteur comprend des parois supplémentaires (56, 58) orthogonales auxdites parois de déviation disposées de façon orthogonale au flux de fluide dans la rainure périmétrique (20) dudit élément de valve tubulaire (13).

10. Connecteur médical selon la revendication 9, **caractérisé en ce que** ledit corps (51) dispose d'une surface de connexion arquée (69, 89) à sa première extrémité (52) et entre les extrémités (70, 71) des parois supplémentaires (56, 58) perpendiculaires aux parois de déviation (55, 57) orthogonales au flux de fluide dans la rainure périmétrique (20) de l'élément de valve tubulaire (13).

11. Connecteur médical selon la revendication 10, **caractérisé en ce que** ladite surface arquée (69) à la première extrémité (52) du corps (51) du déflecteur (50) peut coopérer avec les parois d'extrémité de la rainure périmétrique (20) de l'élément de valve tubulaire (13), ladite coopération générant une force sur ledit corps (51) qui peut le déplacer de manière élastique à l'intérieur de la deuxième partie de connexion (11), éloignant le corps ou le faisant sortir de la cavité (6) de la partie centrale tubulaire (3) du corps du connecteur (2).

12. Connecteur médical selon la revendication 10, **caractérisé en ce que** ladite surface arquée (89) à la première extrémité (52) du corps (51) du déflecteur (50) peut coopérer avec des bossages (108) présents à l'intérieur de la rainure périmétrique (20) de l'élément de valve tubulaire (13).

13. Connecteur médical selon la revendication 7, **caractérisé en ce que** les parois de déviation (55, 57) disposées de façon orthogonale au flux de fluide dans la rainure périmétrique (20) dudit élément de valve tubulaire (13) présentent une zone triangulaire (80) à la deuxième extrémité (53) du corps du déflecteur.
